# EUROPEAN PATENT SPECIFICATION

(11) **EP 1 601 786 B1**
(45) Date of publication and mention of the grant of the patent: **29.08.2007**
(21) Application number: 03768003.0
(22) Date of filing: 16.12.2003
(51) Int. Cl.: C12Q 1/68, G01N 33/53

(54) **Tagged polyfunctional reagents capable of reversibly binding target substances in a pH-dependent manner**
Markierte polyfunktionale Reagenzien, die in der Lage sind, Zielmoleküle pH-abhängig reversibel zu binden
Réactifs polyfonctionnels marqués capables de liaison reversible pH-dépendante à des substances cibles

(30) Priority: 16.12.2002 GB 0229287
(43) Date of publication of application: 07.12.2005
(73) Proprietor: INVITROGEN CORPORATION, Carlsbad, CA 92008 (US)
(72) Inventor: BAKER, Matthew, Maidstone, Kent ME15 9UJ (GB); DOUGLAS, Simon, Knutsford, Cheshire WA16 8AE (GB); LAWRENCE, Elliot, Maidstone, Kent ME16 8LL (GB)
(74) Representative: Williams, Richard Andrew Norman
(86) International application number: PCT/GB2003/005496
(87) International publication number: WO 2004/055213

(56) References cited:
- EP-A- 0 707 077
- WO-A-02/48164
- WO-A-99/22020
- WO-A-99/29703
- US-A- 4 435 509
- US-A- 4 772 550
- US-A- 4 952 519
- US-A- 5 030 697
- US-A- 5 616 467
- US-A- 5 762 903
- US-A- 5 958 788
- US-B1- 6 310 199
- GARRET-FLAUDY F ET AL: "Use of the avidin (imino)biotin system as a general approach to affinity precipitation" BIOTECHNOLOGY AND BIOENGINEERING. INCLUDING: SYMPOSIUM BIOTECHNOLOGY IN ENERGY PRODUCTION AND CONSERVATION, JOHN WILEY & SONS. NEW YORK, US, vol. 71, no. 3, 2000, pages 223-234, XP002226762 ISSN: 0006-3592

## Description

### Field of the Invention

The present invention relates to polyfunctional reagents, and in particular to reagents that are capable of binding to target substances and comprise a member of a specific binding pair which allows the bound target substance to be further manipulated, and optionally detected. The present invention further relates to methods of using and kits comprising the polyfunctional reagents.

### Background of the Invention

Many methods for the extraction of nucleic acid are known including the use of phenol/chloroform, salting out, chaotropic salts and silica resins, affinity resins, ion exchange chromatography and magnetic beads, see for example US Patent Nos: 5,057,426 and 4,923,978, EP 0 512 767 A and EP 0 515 484 A and WO 95/13368, WO 97/10331 and WO 96/18731. These methods suffer from a variety of disadvantages in that the reagents and conditions they employ are often toxic and contaminate nucleic acid samples or the methods involve harsh conditions that denature the target nucleic acid.

EP 0 707 077 A (Johnson & Johnson) describes a synthetic water soluble polymer formed by addition polymerisation of an ethylenically unsaturated monomer having an amine group and its use to precipitate nucleic acids at acid pH and release at alkaline pH. It suggests using the polymer in a water soluble free form or attached to a water insoluble substrate such as an affinity column or polymeric, glass or other inorganic particles. The method disclosed in this application suffers from the disadvantage that the release of nucleic acids is performed at extremes of pH, at high temperature and/or high salt concentrations where the nucleic acids, especially RNA, can become denatured, degraded or require further purification or adjustments before storage and analysis. By way of example, nucleic acid is bound at pH2.3 and released by sodium hydroxide and boiling for 10 minutes at 100°C.

WO 99/29703 (DNA Research Instruments Limited) discloses "charge switch" materials that are capable of reversibly binding nucleic acid at a first pH and then releasing it at a second, higher pH, where the conditions used to release the nucleic acid are mild and do not require the use of extremes of pH, heat or the use of toxic reagents. Further materials having charge switching properties are disclosed in WO 02/48164 (DNA Research Innovations Limited), including biological buffers and polymerised forms thereof, such as Bis-Tris (bis-2-hydroxyethyliminotrishydroxymethylmethane) and poly Bis-Tris.

WO 99/29703 (Promega Corporation) discloses the use of solid phases for purifying nucleic acid which bind nucleic acid in a sample at a low pH and releasing the nucleic acid at a higher pH. The application exemplifies the use of solid phases incorporating histidine or polyhistidine groups. The nucleic acid binding materials are covalently linked to solid phases such as magnetic particles and there is no disclosure of the materials being further derivatised.

### Summary of the Invention

Broadly, the present invention relates to polyfunctional reagents that are capable of reversibly binding to a target substance, wherein the reagents further comprise a member of a specific binding pair for manipulating and/or detecting the target substance when bound to the polyfunctional reagent. In particular, the present invention relates to the binding of target substances such as nucleic acid, proteins, polypeptides, cells, cell components, microorganisms or viruses using a charge switch compound which is capable of binding the target substance at a first pH and then releasing it at a second pH, usually higher than the first. Charge switch compounds are described in WO 99/29703 and WO 02/48164. In some aspects, the polyfunctional reagents are soluble, e.g. water soluble. As indicated herein, in other embodiments, the member of a specific binding pair of the polyfunctional reagent may be employed to bind it to a solid phase.

Accordingly, in one aspect, the present invention provides a soluble polyfunctional reagent which is capable at a first pH of binding to a target substance and is capable at a second pH of releasing the target substance, wherein the reagent further comprises a member of a specific binding pair which is capable of binding to a specific binding partner for manipulating or detecting the target substance when bound to the polyfunctional reagent.

Thus, the present invention provides a way of manipulating or detecting target substances which are bound to charge switch materials. As discussed further below, in preferred embodiments, the reagents are water soluble polymers formed from two or more monomeric units by addition, condensation or cross-linking that are capable of reversibly binding to target substance and especially nucleic acid. This enables the polymers to bind to the target substance in the liquid phase where the binding kinetics are usually superior, and then the complex of the reagent and the target substance can be detected and/or manipulated by virtue of one of more members of a specific binding pair linked to the reagent. In a preferred embodiment, the specific binding pair member can be captured on a solid phase on which its binding partner is immobilised and/or is a label that can be directly or indirectly detected. For binding negatively charged target materials such as nucleic acid and some proteins, the first pH at which binding takes places is lower than the second at which the target substance can be released from the polyfunctional reagent. For binding positively charged target materials, the first pH is typically higher than the second, with the target substance released at the second pH by reducing the negative charge on the polyfunctional reagent.

Typically, the polyfunctional reagent will be water soluble. For the avoidance of doubt, in the present invention, the polyfunctional reagent are generally soluble when added to systems, but may become insoluble upon binding to the target substance. By way of example, some of the polymeric materials disclosed herein precipitate on binding to nucleic acid. As indicated above, the member of a specific binding pair of the polyfunctional reagent may also be used as a way of immobilising the reagent on a support prior to contact with a sample containing the target substance.

In a further aspect, the present invention provides a kit comprising a polyfunctional reagent as defined herein, optionally in combination with one or more other components. In particular, the kit may comprise a solid phase having the binding partner of the specific binding member immobilised thereon. For example, magnetic or paramagnetic beads may be coated with streptavidin to bind to biotin labelled poly-tris polyfunctional reagent.

In a further aspect, the present invention provides a method employing a soluble polyfunctional reagent as described herein, wherein the method comprises:
contacting a sample containing a target substance with the polyfunctional reagent under conditions where the target substance binds to the polyfunctional reagent; and
manipulating or detecting the reagent and/or the bound target substance by employing the specific binding member of the polyfunctional reagent.

In preferred embodiments, the target substance is nucleic acid. In the present application, nucleic acid includes single or double stranded DNA, RNA or oligonucleotides. It includes non-genomic nucleic acid, such as cellular vector DNA or RNA, self-replicating satellite nucleic acids or plasmid DNA, and genomic nucleic acids, such as host cell chromosomes and ribosomal RNA. As nucleic acid is negatively charged, it can be bound by the reagent at a first pH at which the charge switch portion of the reagent is positively charged and then released at a second, higher pH at which the reagent is less positive, neutral or negatively charged. This is discussed in more detail in the section on charge switch materials below.

However, in other embodiments, the target substance may be a protein, a polypeptide, a cell or a component of a cell, a lipid, a carbohydrate, a virus or a microorganism. The present invention can be used to bind target substances that are negatively charged, such as nucleic acid, some polypeptides and cells, or substances that are positively charged, for example polypeptides such as histones or lysozyme or a virus particle, some of which have a net positive charge. By way of example, these materials could be bound using a polyacrylic acid polymer to bind the substance around a neutral pH and then releasing the substance by reducing the pH, e.g. to below pH 4 to reduce the charge on the polyfunctional reagent.

Preferably, the reagent includes a plurality of specific binding pair members to increase the affinity of the interaction between the reagent and any binding partner or to increase a signal from label groups, to facilitate detection.

In the present invention, the specific binding pair member may be for detecting the reagent and any bound target material includes detecting labels directly or indirectly. The labels include:
(1) Fluorescent labels, such a fluorescein isothiocyanate, or combinations of labels to provide acceptor and donor (FRET) systems or polarised fluorescence systems.
(2) Enzyme labels which act, directly or indirectly, on a substrate to produce a detectable result, e.g. horse radish peroxidase or alkaline phosphatase.
(3) Chemiluminescent labels such as Luninol.
(4) Radioactive labels such as Iodine-125.
(5) Colorometric compounds such as dyes, e.g. Cibacron Blue, or coloured latex particles.
(6) Agglutination labels that cause changes in light scattering, e.g. gold sols.

Thus, in one embodiment, the present invention provides a method of generically labelling target substances, and especially nucleic acid, by contacting the target substance with a polyfunctional reagent comprising a member of a specific binding pair wich is a label so that the polyfunctional reagent binds to the target substance and detecting the polyfunctional reagent/target substance complex using the label.

In the present invention, the term "specific binding pair" is used to describe a pair of molecules comprising a specific binding member (sbm) and a binding partner (bp) which have particular specificity for each other and which in normal conditions bind to each other in preference to binding to other molecules. The interaction of the specific binding pair is typically non covalent. The term "specific binding pair" is also applicable where either or both of the specific binding member and binding partner comprise just the binding part of a larger molecule.

Examples of a specific binding pair include an antibody and an antigen, a label and an antibody capable of binding the label, biotin and avidin or streptavidin, a ligand and a receptor, a lectin and a carbohydrate, an enzyme and a cofactor or substrate, a bacteriophage binding to microbial cell walls or a component thereof, cells binding via receptors or antibodies or lectins, oppositely charged ionic groups, redox/electrochemical groups, a chelating group and its binding partner, two hydrophobic substances that are capable of binding to a each other in an aqueous system such as a dye, a phenyl, aliphatic chains, cyclic dextrans, fatty acids, a nucleic acid intercalating group and nucleic acid, or a nucleic acid molecule capable of hybridising to a complementary nucleic acid sequence, including mRNA, polydT, RNA, PNA, primers, oligonucleotides and other polynucleotide interactions.

Preferred specific examples of specific binding pairs include biotin and avidin, biotin and streptavidin, fluorescein isothiocyanate (FITC) and an anti-FITC antibody, an anti-digoxygenin antibody and digoxygenin, maltose binding protein and maltose, glutathione binding to GST, an ethidium bromide or Cy3 intercalating group and nucleic acid, Ni²⁺ ion and polyhistidine (e.g. hexa-His) and Concanavalin A binding to sugar residues. The Sigma catalogue 2000-2001 on page 1922 provides examples of specific binding pairs of reagents.

The member of the specific binding pair permits the reagent and any bound target materials to be manipulated, for example allowing the target substance to be separated from a mixture where it is present with other materials, by contacting the target substance with a solid phase on which the binding partner of the specific binding member has been immobilised so that the specific binding pair bind the functionalised reagent and any bound target substance to the solid phase, thereby allowing it to be separated from the mixture.

By way of example, a functionalised reagent has one or more biotin or avidin/streptavidin groups and can be contacted with a mixture of nucleic acid and other materials (e.g. resulting from lysing cells) and then the bound nucleic acid can be separated using a solid phase on which its binding partner is immobilised. After binding the nucleic acid can be released into solution (e.g. into PCR or storage buffer) by changing the pH. Alternatively, the solid phase with the target substance can be added directly to a reaction mixture, e.g. DNA on a bead can be added to a PCR reaction directly.

In an alternative, preferred embodiment, the polyfunctional reagent comprises a detectable label that is also a specific binding member capable of binding to a binding partner immobilised on a solid phase. An illustrative example of this is provided in example 1 in which the polyfunctional reagent is polyTris labelled with FITC as the isothiocyanate groups of the FITC are reactive towards the hydroxyl groups of the poly-Tris. This reagent is capable of binding to anti-fluorescein antibodies immobilised on a solid phase.

In other embodiments, polyfunctional reagents having one or more tagging groups which are labels and members of a specific binding pair may be employed as separation tags, e.g. using a solid phase such as a bead having the binding partner immobilised thereon. For example, a system employing a tagging group which is a fluorescent label (e.g. FITC) could be used in conjunction with beads on which anti-label antibody was linked to separate nucleic acid binding to the polyfunctional reagent by means of a conventional Fluorescent Activated Cell Sorter (FACS machine).

In embodiments of the invention in which a solid phase is employed, e.g. to capture the polyfunctional reagent, the solid phase may be a magnetisable material, a tube, a well, a tip, a probe, a pipette, a membrane, a filter, a bead, a particle, a sheet, a slide, a plug. The solid phase can be formed from glass, silica, plastic, a mineral, a carbohydrate, paper, or a natural product such as cellulose, and combinations thereof.

Preferably, the portion of the polyfunctional reagent which is capable of binding the target substance is a polymer, although this could include the use of dimeric or oligomeric reagents. Examples of suitable polymers are discussed below and include reagents which are a polyhydroxylated amine, a polymerised biological buffer, or a polymerised amino acids. Preferred examples of these types of reagents include poly Bis-Tris or poly-Tris, polyhistidine, or polyhydroxlyated amines which are aliphatic, cyclic or branched, or chitosans, or triethanolamines.

Other preferred types of polymeric polyfunctional reagents comprise a mixed charge polymer, that is an amine group surrounded by COOH groups would provide the right pK value, polyamine compounds such as a polyethylimine (PEI), poly DEAE or a poly quaternary nitrogen group, polyheterocylic or polyaromatic compounds such as polyimidazole or polypyridine.

The skilled person can prepare these materials based on the teaching in this application, the applications referred to herein (especially WO 99/29703 and WO 02/48164) and their common general knowledge in the art.

By way of example, in a 1-Step polymerisation reaction, monomers can be cross linked, e.g. cross linked Bis Tris, or formed by addition reactions such as polymerisation of vinyl monomers with appropriate functional groups included in the one step polymerisation or added later.

Alternatively, a 2 or more step polymerisation reaction can be used in which a back bone polymer is formed, followed by addition of a pendant group (s), e.g. a backbone of polyacrylic acid, polyacylic amine, polyvinyl alcohol, dextran, polyamide, on which Bis Tris or poly-Bis Tris groups are linked as the pendant group.

Additionally, the other functional group can be added simultaneously or after addition of the first group as described in the example below:
By way of example and not limitation, embodiments of the present invention will now be described in more detail with reference to the accompanying figures.

### Brief Description of the Figures

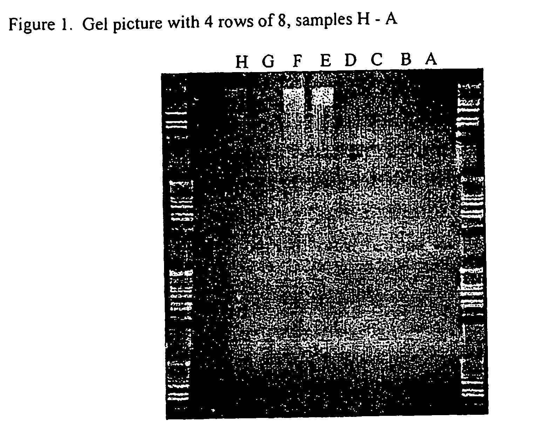

Figure 1 shows a gel demonstrating that a PolyTris-FITC conjugate polyfunctional reagent can be used to specifically bind to nucleic acid.

### Detailed Description

Charge switch materials are described in WO 99/29703 and WO 02/48164 and many of these materials, in particular the water soluble polymers and biological buffers, can be adapted to include tagging groups so that they can be used in accordance with the present invention. Charge switch materials can be used for binding nucleic acid present in a sample by contacting the sample with the charge switch material at a first pH at which the charge switch material has a positive charge and will bind negatively charged nucleic acid, and then releasing the nucleic acid at a second, higher pH at which the charge switch material possesses a neutral, negative or less positive charge than at the first pH. In alternative embodiments, charge switch materials can also be used to bind positively charged target substances, in this case binding them at a first pH and then releasing the substances at a second, lower pH at which the charge switch material is neutral, positive or less negative than the first pH.

Generally the charge switch material will possess an overall positive charge, that is the sum of all positive and negative charges on the charge switch material as a whole is positive. It is possible (though not preferred), however, that the charge switch material as a whole could be negatively charged, but have areas of predominantly positive charge to which the nucleic acid can bind. The change in the charge of the material is referred to herein as "charge switching" and is accomplished by the use of a "charge switch material". The charge switch material comprises an ionisable group, which changes charge to according to the ambient conditions. The charge switch material is chosen so that the pKa of the ionisable group is appropriate to the conditions at which it is desired to bind nucleic acid to and release nucleic acid from the charge switch material. Generally, nucleic acid will be bound to the charge switch material at a pH below or roughly equal to the pKa, when the charge switch material is positively charged, and will be released at a higher pH (usually above the pKa), when the charge switch material is less positively charged, neutral, or negatively charged.

Similarly, in referring to positively and negatively charged target substances, the present invention generally means the net overall charge of the target substance, although in some circumstances, a target substance may have charged regions of an opposite charge to the net charge that can be bound by an appropriate polyfunctional reagent.

The present invention is more particularly directed to the use of charge switch materials which allow binding and/or releasing (especially releasing) of the nucleic acid to occur under mild conditions of temperature and/or pH and/or ionic strength.

Generally the charge switch material will change charge because of a change in charge on a positively ionisable group from positive to less positive or neutral, as the pH is increased in a range spanning or close to the pKa of the positively ionisable group. This may also be combined with a change of charge on a negatively ionisable group from neutral or less negative to more negative.

The charge switch material may comprise an ionisable group having a pKa between about 3 and 9. For positively ionisable groups, the pKa is more preferably at least about 4.5, 5.0, 5.5, 6.0 or 6.5 and/or at most about 8.5, 8.0, 7.5 or 7.0. A particularly preferred pKa for a positively ionisable group is between about 5 and 8; even more preferred is a pKa between about 6.0 and 7.0, more preferably between about 6.5 and 7.0. The pKa for negatively ionisable groups is preferably between about 3 (3.0) and 7 (7.0), still more preferably between about 4 and 6, further preferably approximately at the pH at which it is desired to bind nucleic acid.

Materials having more than one pKa value (e.g. having different ionisable groups), or combinations of materials having different pKa values, may also be suitable for use as charge switch materials in accordance with the invention, provided that at a first (lower) pH the material(s) possess(es) a positive charge and that at a higher pH the charge is less positive, neutral or negative.

Generally a charge switch will be achieved by changing the pH from a value below to a value above the pKa of the or an ionisable group. However, it will be appreciated that when the pH is the same as the pKa value of a particular ionisable group, 50% of the individual ionisable groups will be charged and 50% neutral. Therefore, charge switch effects can also be achieved by changing the pH in a range close to, but not spanning, the pKa of an ionisable group. For example, at the pKa of a negatively ionisable group, such as a carboxy group (pKa typically around 4), 50% of such groups will be in the ionised form (e.g. COO-) and 50% in the neutral form (e.g. COOH). As the pH increases, an increasing proportion of the groups will be in the negative form.

Preferably the binding step is carried out at a pH of below the pKa of the ionisable group, or (though this is not preferred) within about 1 pH unit above the pKa. Generally the releasing step is carried out at a pH above the pKa of the ionisable group, preferably at a pH between 1 and 3 pH units above the pKa.

The use of strong bases, or weak bases in combination with heating, again as in EP 0 707 077 A, can also lead to degradation of RNA (especially at pH values of 10 or above), and denaturation of double stranded DNA (i.e. irreversible conversion of DNA from the double stranded form at least partially into the single stranded form), which can lead to a lack of specific binding in PCR.

The appropriate choice of pKa value(s) in accordance with the invention allows the step of releasing DNA from the solid phase to be performed under mild conditions, unlike in the prior art. As used herein, the term "mild conditions" generally means conditions under which nucleic acid is not denatured and/or not degraded and/or not depurinated, and/or conditions which are substantially physiological.

Preferably the releasing step is performed at a pH of no greater than about pH 10.5, more preferably no greater than about pH 10.0, 9.8, 9.6, 9.4, 9.2, 9.0, 8.9, 8.8, 8.7, 8.6 or 8.5. Depending on the pKa(s) of the charge switch material, the releasing step may even be performed at lower pH values, such as 8.0, 7.5 or 7.0. Preferably the releasing step is carried out in the substantial absence of NaOH, preferably also the substantial absence of other alkali metal hydroxides, more preferably the substantial absence of strong mineral bases. Substantial absence may mean that the concentration is less than 25mM, preferably less than 20mM, more preferably less than 15mM or 10mM.

The desired change in pH can be achieved by altering the ionic strength of the solution and/or the temperature, since pH is dependent on both these factors. However, neither high temperature nor high ionic strength are generally compatible with the desired mild conditions, and accordingly, the change in pH is preferably not achieved by large changes in ionic strength or temperature. Moreover, increasing ionic strength increases competition of charged species with the nucleic acid for binding to the charge switch material, so can assist in releasing the nucleic acid. Small changes of ionic strength are therefore acceptable and may be used in conjunction with the change in pH to release the nucleic acid, preferably within the limits and ranges given below.

Preferably the temperature at which the releasing step performed is no greater than about 70°C, more preferably no greater than about 65°C, 60°C, 55°C, 50°C, 45°C or 40°C. More preferably, such temperatures apply to the entire process. The releasing step, or the entire process, may even be performed at lower temperatures, such as 35°C, 30°C or 25°C.

Furthermore, the releasing step preferably occurs under conditions of low ionic strength, suitably less than 1M or 500 mM, preferably less than 400mM, 300mM, 200mM, 100mM, 75mM, 50mM, 40mM, 30mM, 25mM, 20mM or 15mM. It may even be below 10mM. The ionic strength may be at least about 5mM, more preferably at least about 10mM. More preferably, these ionic strengths also apply to the binding step.

PCR is sensitive to pH and the presence of charged contaminants. In particularly preferred embodiments, the releasing step is performed using reagents suitable for storing nucleic acid (such as a commercially available storage buffer, e.g. 10mM Tris.HCl, pH8.0-8.5, optionally in the presence of 1mM EDTA), or using reagents suitable for use in a procedure to which the nucleic acid is to be subjected (such as a PCR buffer, e.g. 10mM Tris.HCl, 50mM KCl, pH 8.5).

Common previously known nucleic acid extraction processes require a step of diluting the elution product containing nucleic acid, to make the solution suitable for e.g. PCR. Preferably the present invention substantially avoids diluting the released nucleic acid.

Preferably the step of binding DNA occurs under mild conditions, suitably at a pH of no less than 3.0, preferably no less than 3.5, 4.0, 4.5 or 5.0. Previous methods have used high concentrations of chaotropic agents, such as 8M guanidine. Such conditions may not be necessary in the practice of the present invention, in which the binding step preferably occurs in solution having a total concentration of 1M or less. More preferred temperatures and ionic strengths are as detailed above for the releasing step.

The use of such mild conditions for the release of nucleic acid is especially useful for extracting small quantities of nucleic acid, as the extracted DNA or RNA can be added directly to a reaction or storage tube without further purification steps (e.g. steps necessitated by the use of high ion concentrations in prior art methods), and without the need to dilute high ionic strength (as is the case with prior art methods using high ionic strength to elute the nucleic acid). Therefore loss of nucleic acid through changing the container, imperfect recovery during purification steps, degradation, or denaturation, and dilution of small amounts of nucleic acid can be avoided. This is particularly advantageous when a nucleic acid of interest is present in a sample (or is expected to be present) at a low copy number, such as in certain detection and/or amplification methods.

Broadly speaking, preferred chemical species for use as charge switch materials in accordance with the invention comprise a positively ionisable nitrogen atom, and at least one, but preferably more than one, electronegative group (such as a hydroxy, carboxy, carbonyl, phosphate or sulphonic acid group) or double bond (e.g. C=C double bond), which is sufficiently close to the nitrogen atom to lower its pKa. It has been found that such molecules tend to have suitable pKa values for the extraction of nucleic acid under mild conditions according to the present invention. Preferably at least one (but more preferably more than one) electronegative group is separated from the ionisable nitrogen by no more than two atoms (usually carbon atoms). Hydroxyl groups are particularly preferred electronegative groups (particularly when several hydroxyl groups are present, e.g. in polyhydroxyl amines, such as Tris (C(CH₂OH)₃-NH₂) or Bis-Tris (see below)), as they (1) lower the pKa of the nitrogen atom (e.g. amine group, e.g. from about 10 or 11) to a suitable value around neutral (i.e. pKa of about 7), (2) allow the species to remain soluble/hydrophilic above the pKa, when the nitrogen atom of the amine group loses its positive charge, (3) provide a site for covalent linkage to a tagging groups and/or solid substrates, e.g. a polycarboxylated polymer (such as polyacrylic acid), and (4) are uncharged at pH values suitable for the releasing step and at which procedures such as PCR are performed (typically pH 8.5); the presence of charged species can interfere with PCR especially. Especially preferred are chemical species having an ionisable nitrogen atom and at least 2, 3, 4, 5 or 6 hydroxyl groups. Further examples of polyhydroxylated amines are dialcohol amine reagents such as diethanol amine. In one embodiment, silane reagents based on these compounds can be used to attach [HO-(CH₂)ₙ]₂-N- (CH₂)ₘ- moieties, where n and m are selected from 1 to 10, to tagging groups.

Many standard, weakly basic, buffers are ideal chemical species to provide the ionisable groups of charge switch materials, as they have pKa values close to neutral (i.e. 7) .

The polyfunctional reagents of the present invention can be captured on a solid phase using the interaction of a specific binding pair as disclosed herein. One member of the specific binding pair is provided as the tagging group of the polyfunctional reagent and its binding partner can be immobilised on a solid phase so that the solid phase is then capable of binding to the polyfunctional reagent. Solid phases that can be derivatised in this way include beads, particles, tubes, wells, probes, dipsticks, pipette tips, slides, fibers, membranes, papers, celluloses, agaroses, glass or plastics) in a monomeric or polymeric form via adsorption, ionic or covalent interactions, or by covalent attachment of the binding partner to a polymer backbone which is in turn immobilised onto the solid support.

Solid phase materials, especially beads and particles, may be magnetisable, magnetic or paramagnetic. This can aid removal of the solid phase from a solution containing the released nucleic acid, prior to further processing or storage of the nucleic acid.

Preferably the weakly basic buffers are biological buffers, i.e. buffers from the class of buffers commonly used in biological buffer solutions. Examples of biological buffers may be found in commercial chemical catalogues, such as the Sigma catalogue.

Leaching (i.e. transfer from the solid phase into solution in the liquid phase) of chemical species used to provide ionisable groups in ion exchange resins is a virtually inevitable phenomenon to some extent, especially when the species are immobilised on the solid phase by the interaction of the specific binding pair. Such leaching typically causes impurity in the resultant product, which can lead to significant problems, particularly if the resultant product is intended to be used in PCR (and especially when the species are charged). The use of biological buffers to provide the ionisable groups in charge switch materials can avoid this problem, since leaching of such buffers into the liquid phase will generally not significantly affect the nucleic acid, nor any downstream processes such as PCR to which it might be subjected. Indeed, many biological buffers are routinely used in PCR buffers, storage buffers and other buffer solutions.

In a particularly preferred embodiment, the releasing step takes place in a buffer solution containing the same biological buffer that is used in, as or on the charge switch material portion of the polyfunctional reagent.

Examples of suitable biological buffers for use in charge switch materials in accordance with the invention, and their pKa values, are as follows:
N-2-acetamido-2-aminoethanesulfonic acid ‡‡ (ACES), pKa 6.8;
N-2-acetamido-2-iminodiacetic acid ‡‡ (ADA), pKa 6.6;
amino methyl propanediol † (AMP), pKa 8.8;
3-1,1-dimethyl-2-hydroxyethylamino-2-hydroxy propanesulfonic acid † (AMPSO), pKa 9.0;
N,N-bis2-hydroxyethyl-2-aminoethanesulfonic acid †† (BES), pKa 7.1;
N,N-bis-2-hydroxyethylglycine † (BICINE), pKa 8.3;
bis-2-hydroxyethyliminotrishydroxymethylmethane ‡‡ (Bis-Tris), pKa 6.5;
1,3-bistrishydroxymethylmethylaminopropane ‡‡ (BIS-TRIS Propane), pKa 6.8;
4-cyclohexylamino-1-butane sulfonic acid (CABS), pKa 10.7;
3-cyclohexylamino-i-propane sulfonic acid (CAPS), pKa 10.4;
3-cyclohexylamino-2-hydroxy-1-propane sulfonic acid (CAPSO), pKa 9.6;
2-N-cyclohexylaminoethanesulfonic acid (CHES) pKa 9.6;
3-N,N-bis-2-hydroxyethylamino-2-hydroxypropanesulfonic acid †† (DIPSO), pKa 7.6;
N-2-hydroxyethylpiperazine-N-3-propanesulfonic acid †† (EPPS or HEPPS), pKa 8.0;
N-2-hydroxyethylpiperazine-N-4-butanesulfonic acid t (HEPBS), pKa 8.3;
N-2-hydroxyethylpiperazine-N-2-ethanesulfonic acid †† (HEPES), pKa 7.5;
N-2-hydroxyethylpiperazine-N-2-propanesulfonic acid †† (HEPPSO), pKa 7.8;
2-N-morpholinoethanesulfonic acid ‡ (MES), pKa 6.1;
4-N-morpholinobutanesulfonic acid †† (MOBS), pKa 7.6;
3-N-morpholinopropanesulfonic acid ††(MOPS), pKa 7.2;
3-N-morpholino-2-hydroxypropanesulfonic acid ‡‡ (MOPSO), pKa 6.9;
piperazine-N-N-bis-2-ethanesulfonic acid ‡‡ (PIPES), pKa 6.8;
piperazine-N-N-bis-2-hydroxypropanesulfonic acid †† (POPSO), pKa 7.8;
N-trishydroxymethyl-methyl-4-aminobutanesulfonic acid † (TABS), pKA 8.9;
N-trishydroxymethyl-methyl-3-aminopropanesulfonic acid †† (TAPS), pKa 8.4;
3-N-trishydroxymethyl-methylamino-2-hydroxypropanesulfonic acid †† (TAPSO), pKa 7.4;
N-trishydroxymethyl-methyl-2-aminoethanesulfonic acid †† (TES), pKa 7.4;
N-trishydroxymethylmethylglycine † (TRICINE), pKa 8.1; and
trishydroxymethylaminomethane † (TRIS), pKa 8.1;
histidine*, pKa 6.0, and polyhistidine ‡‡;
imidazole*, pKa 6.9, and derivatives* thereof (i.e. imidazoles), especially derivatives containing hydroxyl groups**;
triethanolamine dimers**, oligomers** and polymers**; and
di/tri/oligo amino acids**, for example Gly-Gly, pKa 8.2; and Ser-Ser, Gly-Gly-Gly, and Ser-Gly, the latter three having pKa values in the range 7-9.

In a preferred embodiment, the buffers marked above with an asterisk (*) are not considered to be biological buffers for the purposes of the invention (whether or not they are designated as such in any chemical catalogue). In a more preferred embodiment, those marked with two asterisks (**) are also not considered to be biological buffers. Preferred biological buffers are marked with a dagger (†), more preferred buffers are marked with two daggers (††), still more preferred buffers are marked with a double dagger (‡) and most preferred buffers are marked with two double daggers (‡‡).

These and other chemical species comprising ionisable groups are typically employed as polymers, preferably following condensation polymerisation).

Biological buffers and other chemical species comprising positively ionisable groups may be used in conjunction with a chemical species containing a negatively ionisable group which has a suitable pKa, preferably in the ranges described above. For example a biological buffer (having one or more positively ionisable nitrogen atoms) may be attached to a polymer or other solid phase material which has exposed carboxy groups even after attachment of the biological buffer. Such a material may bind nucleic acids at a low pH when few of the carboxy groups are negatively charged (i.e. few are in the COO⁻ form, most being in the COOH form) and most of the ionisable nitrogen atoms are positively charged. At higher pH the negative charge is stronger (i.e. a greater proportion of carboxy groups are in the COO⁻ form) and/or the positive charge is weaker, and the nucleic acid is repelled from the solid phase.

Chemical species containing ionisable groups (such as the biological buffers listed above) can be attached to a polymer backbone using known chemistries. For example a chemical species containing a hydroxyl group can be attached using carbodiimide chemistry to a carboxylated polymer backbones. Other chemistries include can be employed by someone skilled in the art using other polymer backbones (e.g. based on polyethylene glycol (PEG) or carbohydrate) using a range of standard coupling chemistries (see e.g. Immobilised Affinity Ligand Techniques, Greg T. Hermanson, A. Krishna Mallia and Paul K. Smith, Academic Press, Inc., San Diego, CA, 1992, ISBN 0123423309, which is incorporated herein by reference in its entirety.)

Alternatively, the chemical species containing ionisable groups can be polymerised without a backbone polymer, using cross-linking agents, for example reagents that couple via a hydroxy group (e.g. carbonyldiimidazole, butanediol diglycidyl ether, dialdehydes, diisothiocyanates). Polymers may also be formed by simple condensation chemistries to generate polymeric amino acids with the appropriate pKa e.g. Gly-Gly.

Preferably such immobilisation, attachment and/or polymerisation of the chemical species containing the ionisable group does not affect the pKa of the ionisable group, or leaves it in the desired ranges given above. For example it is generally preferred not to couple or polymerise the chemical species via a positively ionisable nitrogen atom (in constrast for example to WO97/2982). In the practice of the invention, it is especially preferred to immobilise, attach and/or polymerise the chemical species via an hydroxyl group.

A preferred polymeric material is a dimer or oligomer of Bis-Tris or Tris, or a material formed by attaching a plurality of Bis-Tris or Tris molecules to a polyacrylic acid backbone, e.g. by reacting Bis-Tris or Tris monomer with polyacrylic acid using 1-ethyl-3-dimethylaminopropyl carbodiimide (EDC). The polymer can then be easily separated from the reactants using dialysis against a suitable reagent or water. Preferably the polyacrylic acid has molecular weight of between about 500 and 5 million or more. More preferably it has a molecular weight of between 100,000 and 500,000.

The nature of the resultant Bis-Tris or Tris/polyacrylic acid molecule will depend on the ratio of the coupled components, since the polymer will have different properties depending on the proportion of the acrylic acid groups that are modified with Bis-Tris or Tris, for example it is desirable for some carboxy groups to remain unmodified, as the presence of these will not prevent the Bis-Tris or Tris from binding nucleic acid at low pH (especially if the Bis-Tris or Tris is in excess), but their negative charge at higher pHs will assist with release of the nucleic acid. For use in the present invention, the molar ratio of Bis-Tris or Tris:carboxy groups (before attachment) is preferably between 5:1 and 1:5, more preferably between 3:1 and 1:3, still more preferably between 2:1 and 1:2, further preferably between 1.5:1 and 1:1.5, and most preferably about 1:1.

The presence of high residual charge (i.e. charged species present in solution along with the extracted nucleic acid) may adversely affect the analysis of nucleic acids by PCR, or interfere with the binding of primers, dNTPs or polymerase to the nucleic acid, or to the sequestration of Mg²⁺ ions, which are essential to PCR. It is particularly preferable to avoid residual positive charge.

Preferred materials for use in the invention, such as the biological buffers described above, possess minimal residual positive charge (preferably minimal residual charge) at the pH at which the nucleic acid is released, and/or at pHs 8-8.5, making interference with or inhibition of downstream processes unlikely.

Further examples of charge switching molecules for nucleic acid purification are based on detergents or surfactants that have a hydrophobic portion and a hydrophilic portion which comprises a positively ionisable group with a suitable pKa, e.g. decyl methyl imidazole or dodecyl-Bis-Tris. These detergents/surfactants can be adsorbed onto surfaces e.g. plastic via their hydrophobic portions and the hydrophilic (ionisable) portions can be used to capture nucleic acid.

Another family of suitable materials for capture and easy release of nucleic acids are carbohydrates e.g. glucosamine, polyglucosamine (including chitosans), kanamycins and their derivatives, i.e. sugar ring based structures containing one or more nitrogen atoms surrounded by hydroxyl groups which may also contain other groups such as acetate or sulphate groups to provide a suitable pKa for binding and release of nucleic acids.

Another group of materials with suitable pKa values are nucleic acid bases, e.g. cytidine (pKa 4.2). These can be immobilised via hydroxy groups to a polymer or solid phase carboxy group using carbodiimides.

A still further group of materials having members with suitable pKa values are heterocyclic nitrogen-containing compounds. Such compounds may be aromatic or aliphatic and may be monomers, oligomers or polymers, such as morpholine-, pyrrole-, pyrrolidine-, pyridine-, pyridinol-, pyridone-, pyrroline-, pyrazole-, pyridazine-, pyrazine-, piperidone-, piperidine-, or piperazine-containing compounds, e.g. polyvinylpyridine. Such compounds may be substituted with electronegative groups to bring the pKa value(s) of the ionisable nitrogen atom(s) into an acceptable range, e.g. as defined above. However, in some compounds this may not be necessary, the pKa already being in such a range.

A still further group of charge switch materials for binding nucleic acid have surface amine groups, and in particular amine groups which are not polyamines. These monoamine groups can be represented by the formula -NR₁R₂, where R₁ and R₂ are hydrogen or substituted or unsubstituted alkyl. Although these materials typically have pKa values which at higher than those of materials used in preferred embodiments of the invention, they can be employed in the extracting of nucleic acid, optionally employing them with negatively charged species as described herein to modify the overall pKa of the charge switch material.

A further group are materials that provide ionisable groups capable of acting as charge switch materials and binding nucleic acid are dyes, and in particular biological dyes having pKas between 5 and 8.

Preferred materials for use in accordance with the invention are hydrophilic, for example comprising charge switch materials which are (or which comprise chemical species which before immobilisation or polymerisation are) water soluble.

Once a suitable charge switch material has been prepared, repeated capture and release of nucleic acids can be performed by adjusting the pH up or down. Thus sequential reactions or analysis can be performed on the nucleic acids using the same charge switch material. For example, DNA can be isolated from a biological sample using a PCR tube comprising a charge switch material. Then, following PCR, the amplified DNA product may be isolated from the buffer constituents or primers by adjusting the pH in the same tube.

The compositions and methods of the present invention can be used to separate single stranded RNA or DNA from double stranded DNA, because of the different charge densities on single and double stranded molecules, by appropriate manipulation of the pH or salt concentration. Typically, single stranded molecules will be released from binding to the charge switch material at a lower pH than double stranded molecules.

In some circumstances, for example for the construction of gene chips, and for the preparation of probes, it may be desirable to produce single stranded DNA.
Manipulation of pH and/or ionic strength can assist in purification and release of single stranded nucleic acid. The method of the invention may comprise a prior step of converting double stranded nucleic acid in the sample to single stranded nucleic acid (preferably using a strong base, e.g. 100mM NaOH, or a weak base at high temperature, e.g. 60-100°C). The charge switch is preferably then added simultaneously with a buffer which changes the pH of the sample to the pH for binding single stranded nucleic acid (typically a pH of 4-7). In an alternative embodiment, ssDNA could be obtained by binding dsDNA to the polyfunctional reagent, immobilising the reagent on a solid phase though the interaction of a specific binding pair and then using heat to denature the dsDNA to form ssDNA. This approach would be particularly useful to provide ssDNA for use in an assay for infectious disease.

The methods of the invention preferably include one or more washing steps between the binding and releasing steps. Such (a) washing step(s) will generally be carried out at said first pH, or a pH above said first pH but lower then said second pH, such that the nucleic acid is substantially not released during the washing step(s).

As has been indicated previously, the methods of the invention are particularly suitable for extracting nucleic acid which is then stored or further processed (e.g. by PCR), particularly when the charge switch material is in the form of e.g. a tube or well in which such storage and/or processing can occur. For the avoidance of doubt, however, it is emphasized that the releasing step and any subsequent storage or processing need not be carried out as discrete steps, but can coincide, when said storage or processing occurs at a pH at which release of the nucleic acid occurs. For example, the method of the invention includes binding nucleic acid to a charge switch material coated on or otherwise provided by a PCR tube, washing the bound nucleic acid, and then without a separate releasing step commencing the PCR reaction using a PCR buffer which causes release of the nucleic acid.

In a further aspect, the present invention provides novel charge switch materials for use in the methods of the receding aspects. It further comprises the use of such charge switch materials in such methods. All preferred features of the charge switch materials described in above in the context of the methods apply equally and independently to the present aspect of the invention (i.e. preferred combinations of features may be different in relation to this aspect from the preferred combinations in relation to the method aspects).

### Example 1

A mouse monoclonal antibody raised against fluorescein isothiocyanate (FITC) was coated onto 300ul wells of a polystyrene microtitre plate using 0.1M NaHCO3 at an antibody concentration of 4.6ug/ml. After washing in 0.15M NaCl, the plates were ready to use.

To each row of wells DNA was added in a 50mM potassium acetate buffer at pH4. Wells A - D contained DNA at 20ug/ml, wells E - H contained DNA at 100ug/ml. Uncoated wells were used as a control to detect non-specific binding. To every well, doubling dilutions of Poly Tris coupled to FITC were added and incubated for 1 hour at ambient. The Poly Tris polymer was prepared according to DRI patent applications US 09/586,009 or WO 02/48164 then coupled to FITC in a 0.1M NaHCO₃ buffer by mixing FITC with the Poly Tris at a ratio of approximately 1.25mg to 5mg respectively. Following dialysis, the conjugated polymer (PT-FITC) was ready to use.

In certain rows, the PolyTris-FITC conjugate was omitted to estimate non-specific binding of the DNA.

Having captured the DNA at pH4 and washing the wells with water, the DNA could be recovered by adjusting the pH to 8.5 with 100ul 10mM Tris HC1. The gel pictures (Fig 1) and PicoGreen quantitation results (Table 1) indicate specific binding of DNA from the liquid.

### Example 2

This example employed biotin labelled poly Bis-Tris and streptavidin coated plates. Biotin labelled poly Bis-Tris was prepared by mixing Biotin with EDC and an excess of poly Bis-Tris. For example, 1 gram of poly Bis-Tris was mixed with 200 mg of biotin, 160 mg of EDC in 45ml of 0.1M imidazole buffer pH6.5 to give approximate % wt ratios of biotin to PBT of 20%. Following an overnight incubation and exhaustive dialyis, the polymer was ready for use. The streptavidin coated plates were prepared by adding 300ul of streptavidin at about 75ug/ml in 0.1M NaHCO3 with 0.1% glutaldehyde to each well of a black polystyrene microtitre plate. After an overnight incubation, the plate was washed thoroughly with a saline solution and air dried.

To a series of wells, dilutions of the biotin-PBT was added in 10mM Tris HC1 pH8.5 and incubated for 3hours. The plates washed in the same buffer and then treated with a DNA solution. A solution of calf thymus DNA was made up to 17ug/ml in 16mM potassium acetate pH4 and 200ul added to each well. After incubating for 3h at RT, the wells were washed with water and a solution of Picogreen added directly to each well.

**Results for 20% Biotin-Poly Bis-Tris**

| Dilution of Biotin PBT | DNA yield (ng) |
|---|---|
| 1/30 | 15 |
| 1/50 | 15 |
| 1/90 | 15 |
| 1/170 | 12 |
| 1/330 . | 13 |
| No Biotin-PBT | 6 |
| No Biotin-PBT | 6 |
| No Biotin-PBT | 6 |

The results show the presence of the Biotin-PBT has increased the binding capacity for DNA over the non-treated wells and that the streptavidin coated on the plates is capable of binding to the biotinylated portion of the nucleic acid binding reagent. The method is also effective when the DNA from the sample bound to the polyfunctional reagent prior to contact with the solid phase.

### Example 3

This example used biotin labelled poly Bis-Tris and streptavidin coated Tip Plugs. A 30um pore sintered plastic plug was coated with Streptavidin as described above by soaking the plugs for 2 days and then washing away any unbound material. The plug was then washed in a solution of 20%Biotin-PBT in 10mM Tris-HCl pH8.5 by inserting the plug into a 1ml pipette tip and pumping repeatedly. The unbound polymer was then washed away using the same buffer and the Tip Plug was ready for use.

To test the coated plug, 10ug of Lambda DNA was added to 100ul of serum with 1ml of DRI lysis buffer (DRI part No. CO33) and 10ul of proteinase K at 20mg/ml . After an incubation period of 15 minutes with mixing, 100ul of 1.6M potassium acetate and potassium chloride buffer pH4 was added and mixed. This solution was then pumped across the tip plug several times to bind the DNA. The plug was then washed with water and the DNA eluted with 200ul of 10mM Tris-HCl pH8.5 by pumping several times. The eluted DNA was analysed by uv and gel electrophoresis.

**Results 260/280nm**

| | | | | |
|---|---|---|---|---|
| 260nm | 280nm | ratio | yield | Biotin-PBT plug |
| 0.11 | 0.061 | 1.8 | 1.1ug | Biotin-PBT plug |
| 0.05 | 0.04 | 1.25 | 0 | serum only- no DNA |

These results show that the biotin-poly Bis-Tris selectively binds the DNA from biological samples. The low 260/280 ratio of the eluted material from the control indicates that little or no DNA is present and this was confirmed by gel electrophoresis. The method is also effective when the DNA from the sample bound to the polyfunctional reagent prior to contact with the solid phase.

**Table 1. DNA recovered from each well using 100ul of elution buffer at pH 8.5.**

| DNA Yield (ug/ml) | | | | | | | | |
|---|---|---|---|---|---|---|---|---|
| sample | H | G | F | E | D | C | B | A |
| Row 1 Antibody coated. PT-FITC | 4.53 | 4.53 | 19.5 | 16.42 | 1.19 | 0.85 | 1.0 | 0.95 |
| Row 2 Antibody coated. No PT-FITC | 1.4 | 1.7 | 1.25 | 1.82 | 1.23 | 0.84 | 0.86 | 1.08 |
| Row 3 No antibody. PT-FITC | 0.8.7 | 0.6 | 0.93 | 1.17 | 0.77 | 0.34 | 0.81 | 0.43 |
| Row 4 No antibody. No PT-FITC | 0.5 | 0.39 | 0.29 | 0.27 | 0.61 | 0.46 | 0.26 | 0.82 |

## Claims

1. A soluble polyfunctional reagent which is capable at a first pH of binding to a target substance and is capable at a second pH of releasing the target substance, wherein the reagent comprises a member of a specific binding pair which is capable of binding to a specific binding partner for manipulating or detecting the target substance when bound to the polyfunctional reagent.

2. The polyfunctional reagent of claim 1, wherein the reagent is capable of reversibly binding to the target substance at a first pH at which the reagent is positively charged and releasing the nucleic acid at a second, higher pH at which the reagent is less positive, neutral or negatively charged.

3. The polyfunctional reagent of claim 2, wherein the reagent has positively ionisable groups having a pKa between 4.5 and 8.5.

4. The polyfunctional reagent of claim 2 or claim 3, wherein the first pH at which the target substance binds is no less than pH 3.0 and the second pH at which the target substance is released is between pH 7.0 and 9.0.

5. The polyfunctional reagent of any one of the preceding claims, wherein the target substance is nucleic acid.

6. The polyfunctional reagent of claim 5, wherein the nucleic acid is single or double stranded DNA, RNA or oligonucleotides.

7. The polyfunctional reagent of claim 5 or claim 6, wherein the nucleic acid is non-genomic nucleic acid, cellular vector DNA or RNA, self-replicating satellite nucleic acids or plasmid DNA, genomic nucleic acid, host cell chromosomes or ribosomal RNA.

8. The polyfunctional reagent of claim 1, wherein the reagent is capable of reversibly binding to the target substance at a first pH at which the reagent is negatively charged and releasing the nucleic acid at a second, lower pH at which the reagent is less negative, neutral or positively charged.

9. The polyfunctional reagent of claim 8, wherein the reagent has negatively ionisable groups having a pKa between 3.0 and 7.0

10. The polyfunctional reagent of any one of the preceding claims, wherein the target substance is a protein, a polypeptide, a cell or a component of a cell, a lipid, a carbohydrate, a virus or a microorganism.

11. The polyfunctional reagent of any one of the preceding claims, wherein reagent releases the target substance:
(a) substantially in the absence of strong mineral base; and/or
(b) at a temperature which is less than 70°C; and/or
(c) at an ionic strength which is below 100mM.

12. The polyfunctional reagent of any one of the preceding claims, wherein the reagent is a polyhydroxylated amine, a polymerised biological buffer, or a polymerised amino acids.

13. The polyfunctional reagent of claim 12, wherein the reagent is poly Bis-Tris, poly-Tris, polyhistidine, a polyhydroxylated amine, a chitosan, or a triethanolamine.

14. The polyfunctional reagent of any one of the preceding claims, wherein the reagent comprises a plurality of members of the specific binding pair.

15. The polyfunctional reagent of any one of the preceding claims, wherein the member of the specific binding pair further comprises a label.

16. The polyfunctional reagent of any one of the preceding claims, wherein the specific binding pair is an antibody and an antigen, a label and an antibody capable of binding the label, biotin and avidin or streptavidin, a ligand and a receptor, a lectin and a carbohydrate, an enzyme and a cofactor or substrate, a bacteriophage binding to microbial cell walls or a component thereof, cells binding via receptors or antibodies or lectins, oppositely charged ionic groups, redox/electrochemical groups, a chelating group and its binding partner, two hydrophobic substances that are capable of binding to a each other in an aqueous system, a nucleic acid intercalating group and nucleic acid, or a nucleic acid molecule capable of hybridising to a complementary nucleic acid sequence.

17. The polyfunctional reagent of claim 15 or claim 16, wherein the label is a fluorescent label.

18. The polyfunctional reagent of any one of claims 15 to 17, wherein the specific binding pair is biotin and avidin, biotin and streptavidin, fluorescein isothiocyanate (FITC) and an anti-FITC antibody, an anti-digoxygenin antibody and digoxygenin, maltose binding protein and maltose, glutathione binding to GST, an ethidium bromide or Cy3 intercalating group and nucleic acid, Ni²⁺ ion and polyhistidine, or Concanavalin A binding to sugar residues.

19. A kit comprising a polyfunctional reagent of any one of the preceding claims, optionally in combination with a solid phase on which the polyfunctional reagent is bound or is capable of binding.

20. The kit of claim 19, wherein the solid phase is a magnetisable material, a tube, a well, a tip, a probe, a pipette, a membrane, a filter, a bead, a particle, a sheet, a slide, a plug.

21. The kit of claim 20, wherein the solid phase is magnetic or paramagnetic beads.

22. The kit of any one of claims 19 to 21, wherein the solid phase is formed from glass, silica, plastic, a mineral, a carbohydrate, paper, a cellulose or a combination thereof.

23. A method employing a soluble polyfunctional reagent of any one of claims 1 to 18, wherein the method comprises:
contacting a sample containing a target substance with the polyfunctional reagent under conditions such that the target substance binds to the polyfunctional reagent; and
manipulating or detecting the reagent and/or the bound target substance by employing the specific binding member of the polyfunctional reagent.

24. The method of claim 23, wherein manipulating the reagent comprises contacting the polyfunctional reagent binding to the target substance with a solid phase on which the binding partner of the specific binding member has been immobilised so that the specific binding pair bind and separating the polyfunctionalised reagent and any bound target substance.

25. The method of claim 24, wherein the polyfunctional reagent comprises a detectable label that is a specific binding member capable of binding to a binding partner immobilised on a solid phase.

26. The method of claim 25, wherein the polyfunctional reagent comprises a fluorescent label and the binding partner comprises antibodies capable of binding to the fluorescent label immobilised on the solid phase.

27. The method of claim 26, wherein manipulating the reagent comprises separating the target substance binding to the polyfunctional reagent by means of a fluorescent activated cell sorter.

28. A method employing a soluble polyfunctional reagent of any one of claims 1 to 18, wherein the method comprises:
contacting the polyfunctional reagent with a solid phase having immobilised thereon a binding partner of the member of the specific binding pair of the polyfunctional reagent so that the polyfunctional reagent binds to the solid phase by the interaction of the members of the specific binding pair;
contacting a sample containing a target substance with the polyfunctional reagent under conditions where the target substance binds to the polyfunctional reagent.

29. The method of any one of claims 23 to 28, wherein the target substance is nucleic acid.

30. The method of any one of claims 23 to 29, wherein the solid phase is a magnetisable material, a tube, a well, a tip, a probe, a pipette, a membrane, a filter, a bead, a particle, a sheet, a slide, a plug.

31. The method of claim 30, wherein the solid phase is magnetic or paramagnetic beads.

32. The method of any one of claims 23 to 31, wherein the solid phase is formed from glass, silica, plastic, a mineral, a carbohydrate, paper, a cellulose or a combination thereof.

## Patentansprüche

1. Ein lösliches polyfunktionales Reagens, das bei einem ersten pH in der Lage ist, an eine Zielsubstanz zu binden und das bei einem zweiten pH in der Lage ist, die Zielsubstanz freizusetzen, wobei das Reagens ein Element eines spezifischen Bindungspaars umfasst, das in der Lage ist, an einen spezifischen Bindungspartner zwecks Manipulieren oder Detektieren der Zielsubstanz zu binden, wenn diese an das polyfunktionale Reagens gebunden ist.

2. Das polyfunktionale Reagens aus Anspruch 1, wobei das Reagens in der Lage ist, bei einem ersten pH, bei dem das Reagens positiv geladen ist, reversibel an die Zielsubstanz zu binden, und die Nukleinsäure bei einem zweiten, höheren pH, bei dem das Reagens weniger positiv, neutral oder negativ geladen ist, freizusetzen.

3. Das polyfunktionale Reagens aus Anspruch 2, wobei das Reagens positiv ionisierbare Gruppen mit einem pK_{S} zwischen 4,5 und 8,5 aufweist.

4. Das polyfunktionale Reagens aus Anspruch 2 oder Anspruch 3, wobei der erste pH, bei dem die Zielsubstanz bindet, nicht unter pH 3,0 liegt und der zweite pH, bei dem die Zielsubstanz freigesetzt wird, zwischen pH 7,0 und 9,0 liegt.

5. Das polyfunktionale Reagens aus einem der vorhergehenden Ansprüche, wobei es sich bei der Zielsubstanz um Nukleinsäure handelt.

6. Das polyfunktionale Reagens aus Anspruch 5, wobei es sich bei der Nukleinsäure um einzel- oder doppelsträngige DNA, RNA oder Oligonukleotide handelt.

7. Das polyfunktionale Reagens aus Anspruch 5 oder Anspruch 6, wobei es sich bei der Nukleinsäure um nicht-genomische Nukleinsäure, zelluläre Vektor-DNA oder -RNA, selbstreplizierende Satelliten-Nukleinsäuren oder Plasmid-DNA, genomische Nukleinsäure, Wirtszellenchromosome oder ribosomale RNA handelt.

8. Das polyfunktionale Reagens aus Anspruch 1, wobei das Reagens in der Lage ist, bei einem ersten pH, bei dem das Reagens negativ geladen ist, reversibel an die Zielsubstanz zu binden und die Nukleinsäure bei einem zweiten, niedrigeren pH, bei dem das Reagens weniger negativ, neutral oder positiv geladen ist, freizusetzen.

9. Das polyfunktionale Reagens aus Anspruch 8, wobei das Reagens negativ ionisierbare Gruppen mit einem pK_{S} zwischen 3,0 und 7,0 aufweist.

10. Das polyfunktionale Reagens aus einem der vorhergehenden Ansprüche, wobei es sich bei der Zielsubstanz um ein Protein, ein Polypeptid, eine Zelle oder eine Komponente einer Zelle, ein Lipid, ein Kohlenhydrat, einen Virus oder einen Mikroorganismus handelt.

11. Das polyfunktionale Reagens aus einem der vorhergehenden Ansprüche, wobei das Reagens die Zielsubstanz unter folgenden Voraussetzungen freisetzt:
(a) im Wesentlichen in Abwesenheit starker, mineralischer Base; und/oder
(b) bei einer Temperatur von weniger als 70° C; und/ oder
(c) bei einer lonenstärke von weniger als 100 mM.

12. Das polyfunktionale Reagens aus einem der vorhergehenden Ansprüche, wobei es sich bei dem Reagens um ein polyhydroxyliertes Amin, einen polymerisierten biologischen Puffer oder eine polymerisierte Aminosäure handelt.

13. Das polyfunktionale Reagens aus Anspruch 12, wobei es sich bei dem Reagens um Poly-Bis-Tris, Poly-Tris, Polyhistidin, ein polyhydroxyliertes Amin, ein Chitosan oder ein Triethanolamin handelt.

14. Das polyfunktionale Reagens aus einem der vorhergehenden Ansprüche, wobei das Reagens eine Vielzahl an Elementen des spezifischen Bindungspaares umfasst.

15. Das polyfunktionale Reagens aus einem der vorhergehenden Ansprüche, wobei das Element des spezifischen Bindungspaars des Weiteren einen Marker umfasst.

16. Das polyfunktionale Reagens aus einem der vorhergehenden Ansprüche, wobei es sich bei dem spezifischen Bindungspaar um einen Antikörper und ein Antigen, einen Marker und einen Antikörper, der in der Lage ist, den Marker zu binden, Biotin und Avidin oder Streptavidin, einen Liganden und einen Rezeptoren, ein Lektin und ein Kohlenhydrat, ein Enzym und einen Kofaktor oder Substrat, einen Bakteriophagen, der an mikrobielle Zellwände oder eine Komponente davon bindet, Zellen, die mittels Rezeptoren oder Antikörpern oder Lektinen binden, entgegengesetzt geladene ionische Gruppen, Redox- / elektrochemische Gruppen, eine chelatierende Gruppe und ihren Bindungspartner, zwei hydrophobe Substanzen, die in der Lage sind, in einem wässrigen System aneinander zu binden, eine in Nukleinsäure interkalierende Gruppe und Nukleinsäure, oder ein Nukleinsäuremolekül, das in der Lage ist, an eine komplementäre Nukleinsäuresequenz zu hybridisieren, handelt.

17. Das polyfunktionale Reagens aus Anspruch 15 oder Anspruch 16, wobei es sich bei dem Marker um einen fluoreszenten Marker handelt.

18. Das polyfunktionale Reagens aus einem der Ansprüche 15 bis 17, wobei es sich bei dem spezifischen Bindungspaar um Biotin und Avidin, Biotin und Streptavidin, Fluoresceinisothiocyanat (FITC) und einen Anti-FITC-Antikörper, einen Anti-Digoxigenin-Antikörper und Digoxigenin, Maltose bindendes Protein und Maltose, Glutathion, das an GST bindet, ein Ethidiumbromid oder in Cy3 interkalierende Gruppe und Nukleinsäure, Ni²⁺-Ion und Polyhistidin, oder an Zuckerreste bindendes Concanavalin A handelt.

19. Ein Kit, das ein polyfunktionales Reagens aus einem der vorhergehenden Ansprüche umfasst, optional in Kombination mit einer festen Phase, an die das polyfunktionale Reagens gebunden ist oder binden kann.

20. Das Kit aus Anspruch 19, wobei es sich bei der festen Phase um ein magnetisierbares Material, ein Röhrchen, einen Well, eine Spitze, eine Sonde, eine Pipette, eine Membran, einen Filter, ein Kügelchen, einen Partikel, ein Blech, einen Objektträger, einen Zapfen handelt.

21. Das Kit aus Anspruch 20, wobei es sich bei der festen Phase um magnetische oder paramagnetische Kügelchen handelt.

22. Das Kit aus einem der Ansprüche 19 bis 21, wobei die feste Phase aus Glas, Silica, Kunststoff, einem Mineral, einem Kohlenhydrat, Papier, einer Zellulose oder einer Kombination aus diesen gebildet wird.

23. Ein Verfahren, in dem ein lösliches, polyfunktionales Reagens aus einem der Ansprüche 1 bis 18 verwendet wird, wobei das Verfahren folgendes umfasst:
Kontaktieren einer eine Ziefsubstanz enthaltenden Probe mit dem polyfunktionalen Reagens unter solchen Bedingungen, dass die Zielsubstanz an das polyfunktionale Reagens bindet; und
Manipulieren oder Detektieren des Reagens und/oder der gebundenen Zielsubstanz durch Einsetzen des spezifischen Bindungselements des polyfunktionalen Reagens.

24. Das Verfahren aus Anspruch 23, wobei Manipulieren des Reagens das Kontaktieren des an die Zielsubstanz bindenden polyfunktionalen Reagens mit einer festen Phase, auf welcher der Bindungspartner des spezifischen Bindungselements immobilisiert wurde, sodass das spezifische Bindungspaar bindet, und das Trennen des polyfunktionalisierten Reagens und jeder beliebigen gebundenen Zielsubstanz, umfasst.

25. Das Verfahren aus Anspruch 24, wobei das polyfunktionale Reagens einen detektierbaren Marker umfasst, bei dem es sich um ein spezifisches Bindungselement handelt, das in der Lage ist, an einen auf einer festen Phase immobilisierten Bindungspartner zu binden.

26. Das Verfahren aus Anspruch 25, wobei das polyfunktionale Reagens einen fluoreszenten Marker umfasst und der Bindungspartner Antikörper umfasst, die in der Lage sind, an den auf der festen Phase immobilisierten fluoreszenten Marker zu binden.

27. Das Verfahren aus Anspruch 26, wobei Manipulieren des Reagens das Trennen der an das polyfunktionale Reagens bindenden Zielsubstanz durch einen Fluoreszenz-Zellseparator umfasst.

28. Ein Verfahren, in dem ein lösliches polyfunktionales Reagens aus einem der Ansprüche 1 bis 18 verwendet wird, wobei das Verfahren folgendes umfasst:
Kontaktieren des polyfunktionalen Reagens mit einer festen Phase, auf der ein Bindungspartner des Elements des spezifischen Bindungspaars des polyfunktionalen Reagens immobilisiert wurde, sodass das polyfunktionale Reagens durch Wechselwirkung mit den Elementen des spezifischen Bindungspaars an die feste Phase bindet;
Kontaktieren einer eine Zielsubstanz enthaltenden Probe mit dem polyfunktionalen Reagens unter solchen Bedingungen, dass die Zielsubstanz an das polyfunktionale Reagens bindet.

29. Das Verfahren aus einem der Ansprüche 23 bis 28, wobei es sich bei der Zielsubstanz um Nukleinsäure handelt.

30. Das Verfahren aus einem der Ansprüche von 23 bis 29, wobei es sich bei der festen Phase um ein magnetisierbares Material, ein Röhrchen, einen Well, eine Spitze, eine Sonde, eine Pipette, eine Membran, einen Filter, ein Kügelchen, einen Partikel, ein Blech, einen Objektträger, einen Zapfen handelt.

31. Das Verfahren aus Anspruch 30, wobei es sich bei der festen Phase um magnetische oder paramagnetische Kügelchen handelt.

32. Das Verfahren aus einem der Ansprüche 23 bis 31, wobei die feste Phase aus Glas, Silica, Kunststoff, einem Mineral, einem Kohlenhydrat, Papier, einer Zellulose oder einer Kombination aus diesen gebildet wird.

## Revendications

1. - Réactif polyfonctionnel soluble qui est capable à un premier pH de se lier à une substance cible et est capable à un second pH de libérer la substance cible, le réactif comprenant un élément d'une paire à liaison spécifique qui est capable de se lier à un partenaire de liaison spécifique pour manipuler ou détecter la substance cible lorsqu'elle est liée au réactif polyfonctionnel.

2. - Réactif polyfonctionnel selon la revendication 1, dans lequel le réactif est capable de se lier de façon réversible à la substance cible à un premier pH auquel le réactif est chargé positivement et de libérer l'acide nucléique à un second pH, supérieur, auquel le réactif est moins positivement chargé, neutre ou chargé négativement.

3. - Réactif polyfonctionnel selon la revendication 2, dans lequel le réactif a des groupes ionisables positivement ayant un pKa entre 4,5 et 8,5.

4. - Réactif polyfonctionnel selon la revendication 2 ou la revendication 3, dans lequel le premier pH auquel la substance cible se lie n'est pas inférieur à pH 3,0 et le second pH auquel la substance cible est libérée est entre pH 7,0 et 9,0.

5. - Réactif polyfonctionnel selon l'une quelconque des revendications précédentes, dans lequel la substance cible est un acide nucléique.

6. - Réactif polyfonctionnel selon la revendication 5, dans lequel l'acide nucléique est un ADN, un ARN ou des oligonucléotides simple ou double brin.

7. - Réactif polyfonctionnel selon la revendication 5 ou la revendication 6, dans lequel l'acide nucléique est un acide nucléique non génomique, un ADN ou ARN vecteur cellulaire, des acides nucléiques satellites auto-répliquants ou un ADN plasmidique, un acide nucléique génomique, des chromosomes de cellules hôtes ou un ARN ribosomal.

8. - Réactif polyfonctionnel selon la revendication 1, dans lequel le réactif est capable de se lier de façon réversible à la substance cible à un premier pH auquel le réactif est chargé négativement et de libérer l'acide nucléique à un second pH, inférieur, auquel le réactif est chargé moins négativement, neutre ou chargé positivement.

9. - Réactif polyfonctionnel selon la revendication 8, dans lequel le réactif a des groupes ionisables négativement ayant un pKa entre 3,0 et 7,0.

10. - Réactif polyfonctionnel selon l'une quelconque des revendications précédentes, dans lequel la substance cible est une protéine, un polypeptide, une cellule ou un composant d'une cellule, un lipide, un glucide, un virus ou un microorganisme.

11. - Réactif polyfonctionnel selon l'une quelconque des revendications précédentes, dans lequel le réactif libère la substance cible:
(a) sensiblement en l'absence de base minérale forte; et/ou
(b) à une température qui est inférieure à 70°C ; et/ou
(c) à une force ionique qui est au-dessous de 100 mM.

12. - Réactif polyfonctionnel selon l'une quelconque des revendications précédentes, dans lequel le réactif est une amine polyhydroxylée, un tampon biologique polymérisé ou des acides aminés polymérisés.

13. - Réactif polyfonctionnel selon la revendication 12, dans lequel le réactif est le poly Bis-Tris, le poly-Tris, une polyhistidine, une amine polyhydroxylée, un chitosan ou une triéthanolamine.

14. - Réactif polyfonctionnel selon l'une quelconque des revendications précédentes, dans lequel le réactif comprend une pluralité d'éléments de la paire à liaison spécifique.

15. - Réactif polyfonctionnel selon l'une quelconque des revendications précédentes, dans lequel l'élément de la paire à liaison spécifique comprend en outre un marqueur.

16. - Réactif polyfonctionnel selon l'une quelconque des revendications précédentes, dans lequel la paire à liaison spécifique est un anticorps et un antigène, un marqueur et un anticorps capable de lier le marqueur, la biotine et l'avidine ou la streptavidine, un ligand et un récepteur, une lectine et un glucide, une enzyme et un cofacteur ou substrat, un bactériophage se liant aux parois de cellules microbiennes ou à un composant de celles-ci, des cellules se liant par l'intermédiaire de récepteurs ou d'anticorps ou de lectines, des groupes ioniques chargés de façon opposée, des groupes redox/électrochimiques, un groupe chélatant et son partenaire de liaison, deux substances hydrophobes qui sont capables de se lier l'une à l'autre dans un système aqueux, un groupe d'intercalation entre des acides nucléiques et un acide nucléique ou une molécule d'acide nucléique capable de s'hybrider à une séquence d'acide nucléique complémentaire.

17. - Réactif polyfonctionnel selon la revendication 15 ou la revendication 16, dans lequel le marqueur est un marqueur fluorescent.

18. - Réactif polyfonctionnel selon l'une quelconque des revendications 15 à 17, dans lequel la paire à liaison spécifique est la biotine et l'avidine, la biotine et la streptavidine, l'isothiocyanate de fluorescéine (FITC) et un anticorps anti-FITC, un anticorps anti-digoxygénine et la digoxygénine, une protéine se liant au maltose et le maltose, le glutathion se liant au GST, un groupe d'intercalation bromure d'éthidium ou Cy3 et un acide nucléique, l'ion Ni²⁺ et une polyhistidine, ou la Concanavaline A se liant à des résidus de sucre.

19. - Kit comprenant un réactif polyfonctionnel tel que défini à l'une quelconque des revendications précédentes, facultativement en combinaison avec une phase solide sur laquelle le réactif polyfonctionnel est lié ou est capable de se lier.

20. - Kit selon la revendication 19, dans lequel la phase solide est un matériau magnétisable, un tube, un puits, une pointe, une sonde, une pipette, une membrane, un filtre, une bille, une particule, une feuille, une lame, un bouchon.

21. - Kit selon la revendication 20, dans lequel la phase solide est constituée de billes magnétiques ou paramagnétiques.

22. - Kit selon l'une quelconque des revendications 19 à 21, dans lequel la phase solide est formée à partir de verre, de silice, de matière plastique, d'un minéral, d'un glucide, de papier, d'une cellulose ou d'une combinaison de ceux-ci .

23. - Procédé employant un réactif polyfonctionnel soluble tel que défini à l'une quelconque des revendications 1 à 18, dans lequel le procédé comprend les opérations consistant à :
- mettre en contact un échantillon contenant une substance cible avec le réactif polyfonctionnel dans des conditions telles que la substance cible se lie au réactif polyfonctionnel ; et
- manipuler ou détecter le réactif et/ou la substance cible liée en employant l'élément de liaison spécifique du réactif polyfonctionnel.

24. - Procédé selon la revendication 23, dans lequel la manipulation du réactif comprend la mise en contact du réactif polyfonctionnel se liant à la substance cible avec une phase solide sur laquelle le partenaire de liaison de l'élément de liaison spécifique a été immobilisé de telle sorte que la paire à liaison spécifique se lie et se sépare du réactif polyfonctionnel et de toute substance cible liée.

25. - Procédé selon la revendication 24, dans lequel le réactif polyfonctionnel comprend un marqueur détectable qui est un élément de liaison spécifique capable de se lier à un partenaire de liaison immobilisé sur une phase solide.

26. - Procédé selon la revendication 25, dans lequel le réactif polyfonctionnel comprend un marqueur fluorescent et le partenaire de liaison comprend des anticorps capables de se lier au marqueur fluorescent immobilisé sur la phase solide.

27. - Procédé selon la revendication 26, dans lequel la manipulation du réactif comprend la séparation de la substance cible se liant au réactif polyfonctionnel au moyen d'un trieur de cellules activées fluorescentes.

28. - Procédé employant un réactif polyfonctionnel soluble tel que défini à l'une quelconque des revendications 1 à 18, dans lequel le procédé comprend les opérations consistant à :
- mettre en contact le réactif polyfonctionnel avec une phase solide sur laquelle a été immobilisé un partenaire de liaison de l'élément de la paire à liaison spécifique du réactif polyfonctionnel de telle sorte que le réactif polyfonctionnel se lie à la phase solide par l'interaction des éléments de la paire à liaison spécifique ;
- mettre en contact un échantillon contenant une substance cible avec le réactif polyfonctionnel dans des conditions où la substance cible se lie au réactif polyfonctionnel.

29. - Procédé selon l'une quelconque des revendications 23 à 28, dans lequel la substance cible est un acide nucléique.

30. - Procédé selon l'une quelconque des revendications 23 à 29, dans lequel la phase solide est un matériau magnétisable, un tube, un puits, une pointe, une sonde, une pipette, une membrane, un filtre, une bille, une particule, une feuille, une lame, un bouchon.

31. - Procédé selon la revendication 30, dans lequel la phase solide est constituée de billes magnétiques ou paramagnétiques.

32. - Procédé selon l'une quelconque des revendications 23 à 31, dans lequel la phase solide est formée à partir de verre, de silice, de matière plastique, d'un minéral, d'un glucide, de papier, d'une cellulose ou d'une combinaison de ceux-ci.
